# EUROPEAN PATENT SPECIFICATION

(11) **EP 2 515 881 B1**
(45) Date of publication and mention of the grant of the patent: **02.07.2014**
(21) Application number: 10814747.1
(22) Date of filing: 23.12.2010
(51) Int. Cl.: A61K 9/20, A61K 9/24, A61P 25/24, A61K 31/405

(54) **PROCESS FOR PREPARING TABLETS COMPRISING 5- HYDROXYTRYPTOPHAN AND TRYPTOPHAN**
VERFAHREN ZUR HERSTELLUNG VON TABLETTEN MIT 5-HYDROXYTRYPTOPHAN UND TRYPTOPHAN
PROCEDE DE PREPARATION DE COMPRIMES CONTENANT DU 5-HYDROXYTRYPTOPHANE ET DU TRYPTOPHANE

(30) Priority: 23.12.2009 IT MI20092295
(43) Date of publication of application: 31.10.2012
(73) Proprietor: Ambros Pharma S.r.l., 20122 Milano (IT)
(72) Inventor: STANKOV, Bojidar Mihaylov, I-20146 Milano (IT)
(74) Representative: Cattaneo, Elisabetta
(86) International application number: PCT/IB2010/056053
(87) International publication number: WO 2011/077406

(56) References cited:
- EP-A1- 1 637 185
- GB-A- 1 378 296

## Description

### FIELD OF THE INVENTION

The present invention relates to a process for preparing tablets comprising at least two layers that comprise 5-hydroxytryptophan and tryptophan.

### STATE OF THE ART

5-Hydroxytryptophan (5-HTP) and tryptophan (Trp) are precursors of serotonin and represent a significant approach in the treatment of conditions involving the synthesis of serotonin and its release into the central nervous system. Tryptophan and its metabolites represent alternatives to traditional anti-depressants, but are less invasive than these latter. Pathologies of interest associated with serotonin deficiency in the central nervous system include mood disorders, depression, headaches and diseases associated with eating disorders.

The presence of serotonin in the bloodstream however is not a real treatment for such diseases as serotonin does not cross the blood-brain barrier (BBB). In contrast, tryptophan is transported across the blood-brain barrier by means of a carrier mechanism, while 5-HTP crosses it easily.

It has therefore been proposed to use Trp and 5-HTP in the treatment of diseases associated with serotonin deficiency.

However, to allow such a treatment, Trp and 5-HTP must be administered in very precise amounts in order to avoid their inefficient involvement in serotonin synthesis. It is known that Trp converts to 5-HTP with the result that in the body there is a low concentration of Trp and a high concentration of 5-HTP.

As well as affecting serotonin synthesis, this difference in concentration also has the drawback associated with 5-HTP, which is known to induce gastrointestinal disorders when administered orally. Moreover, the rapid administration of high doses of tryptophan has been shown to make it less bioavailable.

The document EP1637185 describes a formulation for oral administration that has a specific release profile which takes into account the peculiarities of the serotonin synthesis path and the drawbacks associated with the administration of 5-HTP alone.

Specifically, this document describes a two-layered tablet, with one layer termed "fast" and one layer termed "retard", wherein the "fast" layer comprises 5-hydroxytryptophan and the "retard" layer comprises tryptophan or 5-HTP. The release profile of these tablets is controlled, i.e. rapid from the "fast" layer and extended over time from the "retard" layer.

The tablets described in EP1637185 are prepared by separately mixing the ingredients of the "fast" layer and the "retard" layer, which are then loaded separately into the tabletting machine to obtain tablets whose dissolution from the "fast" layer takes place in less than 5 minutes and, from the "retard" layer, between 5 and 7 hours.

Once produced, however, these tablets must have very precise release characteristics following storage periods at ambient temperature exceeding 24 months. Such tablets must in fact exhibit an at least 90% release of the active principle component contained in the "fast" layer within 5 minutes, a 50-70% release of the component contained in the "retard" layer within 4 hours and more than 80% within eight hours. These release specifications must be maintained not only when the tablets are freshly made but also after 3 months and 24 months of storage at ambient temperature.

However, while the tablets produced as in EP1637185 satisfied the specification when freshly made, they did not conform to the required quality at 24 months. In particular, those tablets comprising 5-HTP in the "fast" layer and Trp or 5-HTP in the "retard" layer exhibited a release within 5 minutes from the "fast" layer of about 85% of active principle, and from the "retard" layer a release of about 30-35% within 4 hours and only about 55-60% within 8 hours, at 24 months of storage. The object of the invention is hence to provide tablets which satisfy the specifications of release maintenance even at 24 months of storage.

### SUMMARY OF THE INVENTION

The aforesaid object was achieved by means of a process for preparing tablets which comprises the use of particular ingredients and specific production steps.

In particular, the invention concerns a process for preparing a tablet which comprises at least two layers, wherein one layer is "fast" and comprises 5-hydroxytryptophan (5-HTP) and the other layer is "retard" and comprises tryptophan (Trp) or 5-hydroxytryptophan (5-HTP), said process comprising the following steps:
1. preparing the "fast" layer mixture by means of the following sub-steps:
   i) granulating a mixture of the following ingredients: a first portion of microcrystalline cellulose, a first portion of croscarmellose sodium, 5-hydroxytryptophan (5-HTP), hydroxypropyl methylcellulose and a colorant, with a granulating solution comprising deionized water and polyvinyl pyrrolidone;
   ii) sizing the granulate;
   iii) drying the sized granulate in a vacuum oven at 45°C to a granulate moisture content of less than 1%;
   iv) sieving the dried granulate through an about 2mm mesh;
   v) adding, to the sieved granulate, the remaining portion of croscarmellose sodium, magnesium stearate, dicalcium phosphate and polyvinyl polypyrrolidone, sieved through an about 0.7 mm mesh sieve;
   vi) mixing the product of sub-step v);
2) preparing the "retard" layer mixture by means of the following sub-steps:
   a) mixing one ingredient among tryptophan and 5-hydroxytryptophan with dicalcium phosphate;
   b) granulating the mixture of sub-step a) with a granulating solution comprising deionized water and polyvinyl pyrrolidone;
   c) sizing the granulate of sub-step b) and drying in a vacuum oven at 45°C to a moisture content of less than 1%;
   d) adding, to the previously sieved product of sub-step c), vitamins, talc, magnesium stearate, silicon dioxide, microcrystalline cellulose, polyvinyl polypyrrolidone and hydroxypropyl methylcellulose sieved through an about 0.7 mm mesh sieve; and
4) loading the two mixtures of steps 1) and 2) into a suitable tabletting machine.

In the present invention when reference is made to quantities of an ingredient, use of the expression "a first portion" of a particular ingredient means the use of at least 5% by weight on the total weight of said ingredient, whereas the expression "the remaining portion" of said ingredient refers to the use of a maximum of 95% by weight on the total weight of the same ingredient.

In the present invention use of the term:
- "fast layer" means a tablet layer which releases 90% of active principle within 5 minutes;
- "retard layer" means a tablet layer which releases 50-70% of active principle within 4 hours and more than 80% of active principle within 8 hours.

The invention also proposes to interpose between the fast layer and retard layer a layer which is inert towards the ingredients both of the fast and retard layers. In this regard, the process optionally and advantageously proposes a step 3) of mixing microcrystalline cellulose, dicalcium phosphate, vegetable magnesium stearate and polyvinyl polypyrrolidone, and a step 4) of loading the three mixtures from steps 1), 2) and 3) into a suitable tabletting machine.

The tablets of the invention obtained in this manner exhibit active principle release characteristics, at 24 months of storage at ambient temperature, of more than 90% of 5-HTP from the "fast" layer within 5 minutes of administration, and, from the "retard" layer, about 50-70% of Trp or 5-HTP within 4 hours and more than 80% of Trp or 5-HTP within 8 hours of administration.

Another aspect of the invention concerns a two-layer tablet, wherein one layer is "fast" and comprises the following ingredients:

| Ingredients |
|---|
| 5-HTP |
| microcrystalline cellulose |
| dicalcium phosphate |
| Hydroxypropyl methylcellulose |
| croscarmellose sodium |
| Polyvinyl pyrrolidone |
| vegetable magnesium stearate |
| polyvinyl polypyrrolidone |
| colorant E132 |

and the other layer is "retard" and comprises the following ingredients:

| Ingredients |
|---|
| dicalcium phosphate |
| L-Tryptophan |
| microcrystalline cellulose |
| vitamins |
| hydroxypropyl methylcellulose |
| Polyvinyl pyrrolidone |
| vegetable magnesium stearate |
| silicon dioxide |
| Talc |
| polyvinyl polypyrrolidone |

A further aspect of the invention concerns a two-layer tablet, wherein one layer is "fast" and comprises the following ingredients:

| Ingredients |
|---|
| 5-HTP |
| microcrystalline cellulose |
| dicalcium phosphate |
| hydroxypropyl methylcellulose |
| croscarmellose sodium |
| polyvinyl pyrrolidone |
| vegetable magnesium stearate |
| Polyvinyl polypyrrolidone |
| colorant E132 |

and the other layer is "retard" and comprises the following ingredients:

| Ingredients |
|---|
| dicalcium phosphate |
| 5-HTP |
| microcrystalline cellulose |
| Vitamins |
| Hydroxypropyl methylcellulose |
| Polyvinyl pyrrolidone |
| vegetable magnesium stearate |
| silicon dioxide |
| talc |
| polyvinyl polypyrrolidone |

Another aspect of the invention concerns a three-layer tablet wherein one layer is "fast" and comprises:

| Ingredients |
|---|
| 5-HTP |
| microcrystalline cellulose |
| dicalcium phosphate |
| hydroxypropyl methylcellulose |
| croscarmellose sodium |
| Polyvinyl pyrrolidone |
| vegetable magnesium stearate |
| polyvinyl polypyrrolidone |
| colorant E132 |

one is an inert layer which comprises:

| |
|---|
| microcrystalline cellulose |
| dicalcium phosphate |
| Polyvinyl polypyrrolidone |
| vegetable magnesium stearate |

and one is a "retard" layer which comprises:

| Ingredient |
|---|
| dicalcium phosphate |
| L-tryptophan |
| microcrystalline cellulose |
| polyvinyl polypyrrolidone |
| Vitamins |
| Hydroxypropyl methylcellulose |
| Polyvinyl pyrrolidone |
| vegetable magnesium stearate |
| silicon dioxide |
| talc |

### DETAILED DESCRIPTION OF THE INVENTION

The invention therefore concerns a process for preparing a tablet which comprises at least two layers, wherein one layer is "fast" and comprises 5-hydroxytryptophan (5-HTP) and the other layer is "retard" and comprises tryptophan (Trp) or 5-hydroxytryptophan (5-HTP), said process comprising the following steps:
1) preparing the "fast" layer mixture by means of the following sub-steps:
   i) granulating a mixture of the following ingredients: a first portion of microcrystalline cellulose, a first portion of croscarmellose sodium, 5-hydroxytryptophan (5-HTP), hydroxypropyl methylcellulose and a colorant, with a granulating solution comprising deionized water and polyvinyl pyrrolidone;
   ii) sizing the granulate;
   iii) drying the sized granulate in a vacuum oven at 45°C to a granulate moisture content of less than 1%;
   iv) sieving the dried granulate through an about 2mm mesh;
   v) adding, to the sieved granulate, the remaining portion of croscarmellose sodium, magnesium stearate, dicalcium phosphate and polyvinyl polypyrrolidone, sieved through an about 0.7 mm mesh sieve;
   vi) mixing the product of sub-step v);
2) preparing the "retard" layer mixture by means of the following sub-steps:
   a) mixing one ingredient among tryptophan and 5-hydroxytryptophan with dicalcium phosphate;
   b) granulating the mixture of sub-step a) with a granulating solution comprising deionized water and polyvinyl pyrrolidone;
   c) sizing the granulate of sub-step b) and drying in a vacuum oven at 45°C to a moisture content of less than 1%;
   d) adding, to the previously sieved product of sub-step c), vitamins, talc, magnesium stearate, silicon dioxide, microcrystalline cellulose, polyvinyl polypyrrolidone and hydroxypropyl methylcellulose sieved through an about 0.7 mm mesh; and
4) loading the two mixtures of step 1) and 2) into a suitable tabletting machine. Preferably sub-step 2) a) comprises the use of L-Trp.

The invention also proposes to interpose, between the fast layer and retard layer, a layer which is inert towards the ingredients of both the fast and retard layers. In this regard, the process optionally and advantageously provides a step 3) of mixing microcrystalline cellulose, dicalcium phosphate, vegetable magnesium stearate, and polyvinyl polypyrrolidone and a step 4) of loading the three mixtures from steps 1), 2) and 3) into a suitable tabletting machine.

Suitable vitamins in sub-step 2) d) include nicotinamide (vitamin pp) and pyridoxine hydrochloride (vitamin B6); suitable colorants in sub-step 1) i) include the colorant E 132.

The mixing sub-step vi) is preferably carried out for about 10 minutes.

The step 3) of optionally mixing the inert layer components is preferably carried out for 10 minutes.

The tablets obtained according to the process of the invention have a diameter within the range of 0.5 to 1.2 cm and a suitable shape for oral administration.

The tablets of the invention whether in two layers or in three layers therefore exhibit a release profile in the physiological environment that is within the required storage specifications. In particular, at 24 months the tablets of the invention exhibit an about 98% release of 5-HTP from the "fast" layer within 5 minutes, a 60% release of 5-HTP or Trp from the "retard" layer within 4 hours and a 93.80% release of Trp or 5-HTP from the "retard" layer within 8 hours.

The invention will now be described with reference to working examples by way of non-limiting illustration.

### Experimental part

### Example 1

### 1) Preparation of the "fast" layer

The following ingredients were used in the indicated amounts:

| Ingredient | Percentage by weight (%) |
|---|---|
| 5-HTP | 16.84 |
| microcrystalline cellulose | 55.43 |
| dicalcium phosphate | 16.67 |
| hydroxypropyl methylcellulose | 3.33 |
| croscarmellose sodium | 2.67 |
| Polyvinyl pyrrolidone | 2.00 |
| vegetable magnesium stearate | 1.00 |
| Polyvinyl polypyrrolidone | 2.00 |
| colorant E132 | 0.06 |
| **Total** | **100** |

A mixture, sieved through a 0.7 mm mesh, of 5-HTP, hydroxypropyl methylcellulose, colorant E132, a first portion of microcrystalline cellulose and a first portion of croscarmellose sodium, was mixed in a 4-way blender. The granulate obtained in this manner was sized by means of a Viani basket granulator with 4 mm mesh, then the sized granulate was dried in a vacuum oven at 45°C to a moisture level of less than 1%. The dried product thus obtained was sieved through a 2 mm mesh.

The remaining portion of croscarmellose sodium, the remaining portion of microcrystalline cellulose, magnesium stearate, polyvinyl polypyrrolidone and dicalcium phosphate, previously sieved through a 0.7 mm sieve, were added to the sieved granulate in a 4-way blender and the mixture obtained was mixed for 10 minutes

### 2) Preparation of the "retard" layer

The following ingredients were used in the indicated amounts:

| Ingredient | Percentage by weight (%) |
|---|---|
| dicalcium phosphate | 18.46 |
| L-Tryptophan | 53.19 |
| microcrystalline cellulose | 11.70 |
| nicotinamide | 2.30 |
| hydroxypropyl methylcellulose | 10.43 |
| Polyvinyl pyrrolidone | 1.28 |
| vegetable magnesium stearate | 0.85 |
| silicon dioxide | 0.64 |
| pyridoxine hydrochloride | 0.31 |
| talc | 0.64 |
| Polyvinyl polypyrrolidone | 0.21 |
| **Total** | **100** |

A mixture of tryptophan and dicalcium phosphate, sieved through a 0.7 mm mesh sieve, was mixed in a 4-way blender. The thus blended mixture was granulated with a granulating solution of deionized water and polyvinyl pyrrolidone. The granulate was sized by means of a Viani basket granulator with 4 mm mesh. The sized granulate was then oven dried at 45°C to a moisture level of less than 1%. The dried granulate was then sieved through a 2 mm mesh.

Vitamins, talc, magnesium stearate, polyvinyl polypyrrolidone and hydroxypropyl methylcellulose, previously sieved through a 0.7 mm mesh sieve, were added to the granulate in a 4-way blender. 3) Preparation of the final tablet

The mixtures obtained from 1) and 2) were loaded into a multi-station tabletting machine (MANESTY LP), and tablets having the following parameters were obtained:
Weight: about 770 mg (as total of the two layers: fast 300 mg; retard 470 mg)

### Example 2

### 1) Preparation of the "fast" layer

A fast layer as in example 1 was prepared, using the same ingredients in the same amounts and following the same procedure.

### 2) Preparation of the "retard" layer

The following ingredients were used in the indicated amounts:

| Ingredient | Percentage by weight (%) |
|---|---|
| dicalcium phosphate | 30.66 |
| 5-HTP | 21.47 |
| microcrystalline cellulose | 26.11 |
| nicotinamide | 2.30 |
| hydroxypropyl methylcellulose | 15.47 |
| Polyvinyl pyrrolidone | 1.28 |
| vegetable magnesium stearate | 0.85 |
| silicon dioxide | 0.7 |
| Pyridoxine hydrochloride | 0.31 |
| talc | 0.64 |
| Polyvinyl polypyrrolidone | 0.21 |
| **Total** | **100** |

A mixture of 5-HTP and dicalcium phosphate, sieved through a 0.7 mm mesh sieve, was mixed in a 4-way blender. The thus blended mixture was granulated with a granulating solution of deionized water and polyvinyl pyrrolidone. The granulate was sized by means of a Viani basket granulator with 4 mm mesh. The sized granulate was then oven dried at 45°C to a moisture level of less than 1%. The dried granulate was then sieved through a 2 mm mesh.

Vitamins, talc, magnesium stearate, polyvinyl polypyrrolidone and hydroxypropyl methylcellulose, previously sieved through a 0.7 mm mesh sieve, were added to the granulate in a 4-way blender.

### 3) Preparation of the final tablet

The mixtures obtained from 1) and 2) were loaded into a multi-station tabletting machine (MANESTY LP), and tablets having the following parameters were obtained:
Weight: about 770 mg (as total of the two layers: fast 300 mg; retard 470 mg)

### Example 3: preparation of the three-layer tablet with inert layer

### 1) Preparation of the "fast" layer

A fast layer as in example 1 was prepared, using the same ingredients in the same amounts. The procedure applied was the same as in example 1.

| Ingredient | Percentage by weight (%) |
|---|---|
| 5-HTP | 16.84 |
| microcrystalline cellulose | 55.43 |
| dicalcium phosphate | 16.67 |
| hydroxypropyl methylcellulose | 3.33 |
| croscarmellose sodium | 2.67 |
| Polyvinyl pyrrolidone | 2.00 |
| vegetable magnesium stearate | 1.00 |
| Polyvinyl polypyrrolidone | 2.00 |
| colorant E132 | 0.06 |
| **Total** | **100** |

### 2) Preparation of the "retard" layer

The same ingredients as in example 1 were used in the amounts indicated below and the process described in example 1 was followed.

| Ingredient | Percentage by weight (%) |
|---|---|
| dicalcium phosphate | 11.01 |
| L-Tryptophan | 67.57 |
| microcrystalline cellulose | 3.24 |
| nicotinamide | 2.92 |
| hydroxypropyl methylcellulose | 10.00 |
| Polyvinyl pyrrolidone | 2.43 |
| vegetable magnesium stearate | 0.81 |
| silicon dioxide | 0.81 |
| pyridoxine hydrochloride | 0.40 |
| Polyvinyl polypyrrolidone | 0.1 |
| talc | 0.71 |
| **Total** | **100** |

### 3) Preparation of the inert layer

The following ingredients were mixed for 10 minutes:
microcrystalline cellulose (55% by weight), dicalcium phosphate (11.01% by weight), vegetable magnesium stearate (1% by weight) and polyvinyl polypyrrolidone (1 %).

### 4) Preparation of the final tablet

The mixtures obtained from 1), 2) and 3) were loaded into a multi-station tabletting machine (MANESTY LP), and tablets having the following parameters were obtained:
Weight: about 770 mg (as total of the two layers: fast 300 mg; inert layer 100 mg; retard 370 mg).

The aforesaid three tablets of the invention were evaluated with specific amounts of the essential ingredients indicated in the process of the invention. By following the aforesaid examples and maintaining the weight percentages indicated for the active principles, the two- or three-layer tablets of the invention can be prepared by varying all the said essential ingredients by ± 20%.

### Example 4: Evaluating the storage of the tablet according to example 1 and comparison with the tablet produced as in Example 1 of EP1637185.

Specifically, the prior art tablets were prepared using the following ingredients for the "fast" and "retard" layers and the procedure described in Example 1 of EP1637185.

### Ingredients of the prior art "fast" layer

| Ingredient | Amount by weight (%) |
|---|---|
| 5-Hydroxytryptophan (5-HTP) | 13.29 |
| dicalcium phosphate | 60.53 |
| Microcrystalline cellulose | 24.50 |
| Silicon dioxide | 0.79 |
| Vegetable magnesium stearate | 0.84 |
| Colorant E132 | 0.06 |
| **Total** | **100** |

The colorant was mixed with a portion of microcrystalline cellulose (previously sized through a 0.7 mm sieve). All the ingredients (previously sized through a 0.7 mm sieve) except magnesium stearate were then loaded into a double cone mixer and were mixed for about 10 minutes. At the end of mixing, the magnesium stearate was added and mixing was continued for a further 5 minutes.

### Ingredients of the prior art "retard" layer

| Ingredient | Amount by weight (%) |
|---|---|
| Tryptophan | 64.10 |
| hydroxypropyl methylcellulose | 13.08 |
| dicalcium phosphate | 15.90 |
| Silicon dioxide | 1.03 |
| Vegetable magnesium stearate | 1.79 |
| Talc | 1.03 |
| Vitamin PP | 2.77 |
| Vitamin B6 | 0.31 |
| **Total** | **100** |

All the components (previously sized through a 0.7 mm sieve), except magnesium stearate, were loaded into a double cone mixer and mixed for about 10 minutes. At the end of mixing the magnesium stearate was added and mixing was continued for a further 5 minutes

The mixtures prepared in this manner were fed separately via respective paths into a tabletting machine (MANESTY LP) and the tabletting step was carried out. The tablets obtained had the following parameters:
Weight: about 800 mg (as the total weights of the two layers)

The tablets obtained according to the prior art described in EP1637185 were compared with the tablets obtained as in example 1.

Specifically, the tablets were evaluated for release when freshly prepared and also after storage in their original primary blister packs at ambient temperature for 3 months and 24 months respectively.

In order to meet the storage specifications, the tablets had to exhibit an active principle release of more than 90% within 5 minutes from the "fast" layer, of between 50 and 70% within 4 hours from the "retard" layer and of more than 90% within 8 hours from the "retard" layer.

Table 1 shows the results obtained for the tablet of the invention and Table 2 those for the tablet described in EP1637185.

**Table 1: tablet of the invention**

| Active principle | time | required specification (% release) | result after immediate release (%) | result after 3 months' storage (%) | result at 24 months storage (%) |
|---|---|---|---|---|---|
| 5-THP (fast) | 5 min | >90 | 105.0 | 100.0 | 98.0 |
| Trp (retard) | 4 hours | 50-70 | 68.0 | 68.1 | 62.3 |
| Trp (retard) | 8 hours | >80 | 105.9 | 100.9 | 93.48 |

**Table 2: tablet of the prior art**

| Active principle | time | required specification (% release) | result after immediate release (%) | result after 3 months' storage (%) | result at 24 months storage (%) |
|---|---|---|---|---|---|
| 5-HTP (fast) | 5 min | >90 | 95.0 | 95.0 | 85.0 |
| Trp (retard) | 4 hours | 50-70 | 64.0 | 66.8 | 35.1 |
| Trp (retard) | 8 hours | >80 | 102.0 | 98.2 | 60.2 |

The release of 5-HTP from the "fast" layer at 24 months of storage met the required specifications only in the case of the tablet of the invention, with the prior art tablet proving to be unsatisfactory.

The result for Trp at 24 months of storage was even more evident. In this respect, both 4 hours and 8 hours after administration, the release profile of the prior art tablet differed from that of the tablet of the invention, namely by -27.2% within 4 hours and by -33.3% within 8 hours, these being much lower values than the required specification, hence demonstrating the superiority of the tablet of the invention produced according to the claimed process.

### Example 5: Evaluating the storage of the tablet according to example 2 and comparison with the tablet produced as in Example 1 of EP1637185.

The tablet of the prior art was prepared by following the process as described in example 3, but replacing Trp with 5-HTP in the retard layer, whereas the tablet of the invention was that prepared in example 2.

The tablets obtained according to the prior art and those obtained as in example 2 were subjected to a comparison.

Specifically, the tablets were evaluated for release when freshly prepared and also after storage in their original primary blister packs at ambient temperature for 3 months and 24 months respectively.

In order to meet the storage specifications the tablets had to exhibit an active principle release of more than 90% within 5 minutes from the "fast" layer, of between 50 and 70% within 4 hours from the "retard" layer, and of more than 90% within 8 hours from the "retard" layer.

Table 3 shows the results obtained for the tablet of the invention and Table 4 those for the tablet described in EP1637185.

**Table 3: tablet of the invention of example 2**

| Active principle | time | required specification (% release) | result after immediate release (%) | result after 3 months' storage (%) | result at months' storage (%) |
|---|---|---|---|---|---|
| 5-THP (fast) | 5 min | >90 | 105.8 | 107.6 | 101.9 |
| 5-HTP (retard) | 4 hours | 50-70 | 66.8 | 66.1 | 61.1 |
| 5-HTP (retard) | 8 hours | >80 | 98.2 | 93.5 | 90.2 |

**Table 4: tablet of the prior art**

| Active principle | time | required specification (% release) | result after immediate release (%) | result after 3 months' storage (%) | result at months' storage (%) |
|---|---|---|---|---|---|
| 5-THP (fast) | 5 min | >90 | 95.6 | 96.1 | 87.3 |
| 5-HTP (retard) | 4 hours | 50-70 | 65.2 | 63.8 | 30.2 |
| 5-HTP (retard) | 8 hours | >80 | 98.4 | 84.2 | 50.4 |

The release of 5-HTP from the "fast" layer at 24 months of storage met the required specifications only in the case of the tablet of the invention, with the prior art tablet proving to be unsatisfactory

The result for 5-HTP at 24 months of storage was even more evident. In this respect, both 4 hours and 8 hours after administration, the release profile of the prior art tablet differed from that of the tablet of the invention, namely by -30.9% within 4 hours and by -39.8% within 8 hours, these being much lower values than the required specification hence demonstrating the superiority of the tablet of the invention produced according to the claimed process.

### Example 6: Evaluating the storage of the tablet according to example 3 and comparison with the tablet produced as in Example 1 of EP1637185.

The tablet of the prior art was prepared as indicated in example 1, while the tablet of the invention was that prepared in example 3.

The tablets obtained according to the prior art and those obtained as in example 3 were subjected to a comparison.

Specifically, the tablets were evaluated for release when freshly prepared and also after storage in their original primary blister packs at ambient temperature for 3 months and 24 months respectively.

In order to meet the storage specifications the tablets had to exhibit an active principle release of more than 90% within 5 minutes from the "fast" layer, of between 50 and 70% within 4 hours from the "retard" layer, and of more than 90% within 8 hours from the "retard" layer.

Table 5 shows the results obtained for the tablet of the invention and Table 6 those for the tablet described in EP1637185.

**Table 5: tablet of the invention of example 3**

| Active principle | time | required specification (% release) | result after immediate release (%) | result after 3 months' storage (%) | result at months' storage (%) |
|---|---|---|---|---|---|
| 5-THP (fast) | 5 min | >90 | 108.2 | 103.6 | 100.8 |
| Trp (retard) | 4 hours | 50-70 | 67.6 | 65.3 | 63.7 |
| Trp (retard) | 8 hours | >80 | 102.7 | 98.1 | 94.2 |

**Table 6: tablet of the prior art**

| Active principle | time | required specification (% release) | result after immediate release (%) | result after 3 months' storage (%) | result at 24 months storage (%) |
|---|---|---|---|---|---|
| 5-HTP (fast) | 5 min | >90 | 94.1 | 93.2 | 86.3 |
| Trp (retard) | 4 hours | 50-70 | 66.3 | 63.7 | 33.2 |
| Trp (retard) | 8 hours | >80 | 101.6 | 96.3 | 57.4 |

The release of 5-HTP from the "fast" layer at 24 months of storage met the required specifications only in the case of the tablet of the invention, with the prior art tablet proving to be unsatisfactory.

The result for Trp at 24 months of storage was even more evident. In this respect, both 4 hours and 8 hours after administration, the release profile of the prior art tablet differed from that of the tablet of the invention, namely by -30.5% within 4 hours and by -36.8% within 8 hours, these being much lower values than the required specification, hence demonstrating the superiority of the tablet of the invention produced according to the claimed process, and also that having an inert layer interposed between the fast layer and the retard layer.

## Claims

1. A process for preparing a two-layer tablet, wherein one layer is "fast" and comprises 5-hydroxytryptophan (5-HTP) and the other layer is "retard" and comprises tryptophan (Trp) or 5-hydroxytryptophan (5-HTP), said process comprising the following steps:
1) preparing the "fast" layer mixture by means of the following sub-steps:
i) granulating a mixture of the following ingredients: a first portion of microcrystalline cellulose, a first portion of croscarmellose sodium, 5-hydroxytryptophan (5-HTP), hydroxypropyl methylcellulose and a colorant, with a granulating solution comprising deionized water and polyvinyl pyrrolidone;
ii) sizing the granulate;
iii) drying the sized granulate in a vacuum oven at 45°C to a granulate moisture content of less than 1%;
iv) sieving the dried granulate through an about 2mm mesh;
v) adding, to the sieved granulate, the remaining portion of croscarmellose sodium, magnesium stearate, dicalcium phosphate and polyvinyl polypyrrolidone, sieved through an about 0.7 mm mesh sieve;
vi) mixing the product of sub-step v);
2) preparing the "retard" layer mixture by means of the following sub-steps:
a) mixing one ingredient among tryptophan and 5-hydroxytryptophan with dicalcium phosphate;
b) granulating the mixture of sub-step a) with a granulating solution comprising deionized water and polyvinyl pyrrolidone;
c) sizing the granulate of sub-step b) and drying in a vacuum oven at 45°C to a moisture content of less than 1 %;
d) adding, to the previously sieved product of sub-step c), vitamins, talc, magnesium stearate, silicon dioxide, microcrystalline cellulose, polyvinyl polypyrrolidone and hydroxypropyl methylcellulose sieved through an about 0.7 mm mesh sieve.
4) loading the two mixtures of steps 1) and 2) into a suitable tabletting machine.

2. The process according to claim 1, wherein sub-step 2) a) comprises the use of L-Trp.

3. The process according to claim 1 or 2, wherein a step 3) is proposed of mixing microcrystalline cellulose, dicalcium phosphate, vegetable magnesium stearate and polyvinyl polypyrrolidone, and wherein step 4), in addition to loading the two mixtures from steps 1) and 2) into a suitable tabletting machine, also proposes loading that of step 3) therein.

4. The process according to any one of claims 1 to 3, wherein the colorant of sub-step 1) i) is the colorant E132.

5. The process according to any one of claims 1 to 4, wherein the tablet obtained in step 4) at 24 months of storage exhibits a release of more than 90% of 5-HTP from the "fast" layer within 5 minutes of administration, of about 50-70% of Trp or 5-HTP from the "retard" layer within 4 hours and of more than 80% of Trp or 5-HTP within 8 hours from the "retard" layer.

6. A two-layer tablet, wherein one layer is "fast" and comprises the following ingredients:
| |
|---|
| 5-HTP |
| microcrystalline cellulose |
| dicalcium phosphate |
| Hydroxypropyl methylcellulose |
| croscarmellose sodium |
| Polyvinyl pyrrolidone |
| vegetable magnesium stearate |
| polyvinyl polypyrrolidone |
| colorant E132 |
and the other layer is "retard" and comprises the following ingredients:
| |
|---|
| dicalcium phosphate |
| L-Tryptophan |
| microcrystalline cellulose |
| vitamins |
| hydroxypropyl methylcellulose |
| Polyvinyl pyrrolidone |
| vegetable magnesium stearate |
| silicon dioxide |
| Talc |
| polyvinyl polypyrrolidone |

7. A two-layer tablet, wherein one layer is "fast" and comprises the following ingredients:
| |
|---|
| 5-HTP |
| microcrystalline cellulose |
| dicalcium phosphate |
| Hydroxypropyl methylcellulose |
| croscarmellose sodium |
| Polyvinyl pyrrolidone |
| vegetable magnesium stearate |
| polyvinyl polypyrrolidone |
| colorant E132 |
and the other layer is "retard" and comprises the following ingredients:
| |
|---|
| dicalcium phosphate |
| 5-HTP |
| microcrystalline cellulose |
| vitamins |
| hydroxypropyl methylcellulose |
| Polyvinyl pyrrolidone |
| vegetable magnesium stearate |
| silicon dioxide |
| Talc |
| polyvinyl polypyrrolidone |

8. A three-layer tablet wherein one layer is "fast" and comprises;
| |
|---|
| 5-HTP |
| microcrystalline cellulose |
| dicalcium phosphate |
| hydroxypropyl methylcellulose |
| croscarmellose sodium |
| Polyvinyl pyrrolidone |
| vegetable magnesium stearate |
| polyvinyl polypyrrolidone |
| colorant E132 |
one layer is an inert layer which comprises:
| |
|---|
| microcrystalline cellulose |
| dicalcium phosphate |
| Polyvinyl polypyrrolidone |
| vegetable magnesium stearate |
and one layer is a "retard" layer which comprises:
| |
|---|
| dicalcium phosphate |
| L-tryptophan |
| microcrystalline cellulose |
| polyvinyl polypyrrolidone |
| Vitamins |
| Hydroxypropyl methylcellulose |
| Polyvinyl pyrrolidone |
| vegetable magnesium stearate |
| silicon dioxide |
| talc |

9. A tablet according to any one of claims 6 to 8, for use in the treatment of pathologies or conditions which involve serotonin deficiency.

10. The tablet according to claim 9 wherein the serotonin deficiencies occur in the central nervous system.

## Patentansprüche

1. Verfahren zur Herstellung einer Tablette mit zwei Schichten, worin eine Schicht "schnell" ist und 5-Hydroxytryptophan (5-HTP) umfaßt und die andere Schicht "retardiert" ist und Tryptophan (Trp) oder 5-Hydroxytryptophan (5-HTP) umfaßt, worin das Verfahren die folgenden Schritte umfaßt:
(1) Herstellen der Mischung für die "schnelle" Schicht mittels der folgenden Unterschritte, daß man
(i) eine Mischung aus den folgenden Bestandteilen granuliert: eine erste Portion aus mikrokristalliner Cellulose, eine erste Portion aus Croscarmellose-Natrium, 5-Hydroxytryptophan (5-HTP), Hydroxypropylmethylcellulose und einen Farbstoff mit einer Lösung zum Granulieren, die entionisiertes Wasser und Polyvinylpyrrolidon umfaßt;
(ii) das Granulat klassiert;
(iii) das klassierte Granulat in einem Vakuumofen bei 45 °C auf einen Granulat-Feuchtigkeitsgehalt von weniger als 1 % trocknet;
(iv) das getrocknete Granulat durch ein Sieb mit einer Maschenweite von etwa 2 mm siebt;
(v) dem gesiebten Granulat die verbleibende Portion Croscarmellose-Natrium, Magnesiumstearat, Dicalciumphosphat und Polyvinylpolypyrrolidon, gesiebt durch ein Sieb mit einer Maschenweite von etwa 0,7 mm, zugibt;
(vi) das Produkt aus dem Unterschritt (v) mischt;
(2) Herstellen der Mischung für die "retardierte" Schicht mittels der folgenden Unterschritte, daß man
(a) einen Bestandteil, gewählt aus Tryptophan und 5-Hydroxytryptophan, mit Dicalciumphosphat mischt;
(b) die Mischung aus dem Unterschritt (a) mit einer Lösung zum Granulieren, die entionisiertes Wasser und Polyvinylpyrrolidon umfaßt, granuliert;
(c) das Granulat aus dem Unterschritt (b) klassiert und in einem Vakuumofen bei 45 °C bis auf einen Feuchtigkeitsgehalt von weniger als 1 % trocknet;
(d) dem vorher gesiebten Produkt aus dem Unterschritt (c) Vitamine, Talkum, Magnesiumstearat, Siliciumdioxid, mikrokristalline Cellulose, Polyvinylpolypyrrolidon und Hydroxypropylmethylcellulose, gesiebt durch ein Sieb mit einer Maschenweite von etwa 0,7 mm, zugibt;
(4) die zwei Mischungen aus den Schritten (1) und (2) in eine geeignete Tablettiermaschine lädt.

2. Verfahren nach Anspruch 1, worin der Unterschritt (2) (a) die Verwendung von L-Trp umfaßt.

3. Verfahren nach Anspruch 1 oder 2, worin ein Schritt (3) vorschlagen wird, bei dem man mikrokristalline Cellulose, Dicalciumphosphat, pflanzliches Magnesiumstearat und Polyvinylpolypyrrolidon mischt und worin Schritt (4) zusätzlich zum Einladen der zwei Mischungen aus den Schritten (1) und (2) in eine geeignete Tablettiermaschine auch vorschlägt, diejenige von Schritt (3) darin einzuladen.

4. Verfahren nach einem der Ansprüche 1 bis 3, worin der Farbstoff aus dem Unterschritt (1) (i) der Farbstoff E132 ist.

5. Verfahren nach einem der Ansprüche 1 bis 4, worin die Tablette, die in Unterschritt (4) erhalten wird, nach 24 Monaten Lagerung eine Freisetzung von mehr als 90 % von 5-HTP aus der "schnellen" Schicht innerhalb von 5 Minuten nach der Verabreichung zeigt, etwa 50 bis 70 % Trp oder 5-HTP aus der "retardierten" Schicht innerhalb von 4 Stunden zeigt und mehr als 80 % Trp oder 5-HPT innerhalb von 8 Stunden aus der "retardierten" Schicht zeigt.

6. Tablette mit zwei Schichten, worin eine Schicht "schnell" ist und die folgenden Bestandteile umfaßt:
5-HTP
Mikrokristalline Cellulose
Dicalciumphosphat
Hydroxypropylmethylcellulose
Croscarmellose-Natrium
Polyvinylpyrrolidon
Pflanzliches Magnesiumstearat
Polyvinylpolypyrrolidon
Farbstoff E132;
und die andere Schicht "retardiert" ist und die folgenden Bestandteile umfaßt:
Dicalciumphosphat
L-Tryptophan
Mikrokristalline Cellulose
Vitamine
Hydroxypropylmethylcellulose
Polyvinylpyrrolidon
Pflanzliches Magnesiumstearat
Siliciumdioxid
Talkum
Polyvinylpolypyrrolidon.

7. Tablette mit zwei Schichten, worin eine Schicht "schnell" ist und die folgenden Bestandteile umfaßt:
5-HTP
Mikrokristalline Cellulose
Dicalciumphosphat
Hydroxypropylmethylcellulose
Croscarmellose-Natrium
Polyvinylpyrrolidon
Pflanzliches Magnesiumstearat
Polyvinylpolypyrrolidon
Farbstoff E132;
und die andere Schicht "retardiert" ist und die folgenden Bestandteile umfaßt:
Dicalciumphosphat
5-HTP
Mikrokristalline Cellulose
Vitamine
Hydroxypropylmethylcellulose
Polyvinylpyrrolidon
Pflanzliches Magnesiumstearat
Siliciumdioxid
Talkum
Polyvinylpolypyrrolidon.

8. Tablette mit drei Schichten, worin eine Schicht "schnell" ist und umfaßt:
5-HTP
Mikrokristalline Cellulose
Dicalciumphosphat
Hydroxypropylmethylcellulose
Croscarmellose-Natrium
Polyvinylpyrrolidon
Pflanzliches Magnesiumstearat Polyvinylpolypyrrolidon
Farbstoff E132;
eine Schicht eine inerte Schicht ist, die umfaßt:
Mikrokristalline Cellulose
Dicalciumphosphat
Polyvinylpolypyrrolidon
Pflanzliches Magnesiumstearat;
und eine Schicht eine "retardierte" Schicht ist, die umfaßt:
Dicalciumphosphat
L-Tryptophan
Mikrokristalline Cellulose
Polyvinylpolypyrrolidon
Vitamine
Hydroxypropylmethylcellulose
Polyvinylpyrrolidon
Pflanzliches Magnesiumstearat
Siliciumdioxid
Talkum.

9. Tablette nach einem der Ansprüche 6 bis 8, zur Verwendung bei der Behandlung von Pathologien oder Zuständen, die einen Serotonin-Mangel einschließen.

10. Tablette nach Anspruch 9, worin die Serotonin-Mängel im zentralen Nervensystem auftreten.

## Revendications

1. Procédé de préparation d'un comprimé à deux couches, dans lequel une couche est "rapide" et comprend du 5-hydroxytryptophane (5-HTP) et l'autre couche est "retard" et comprend du tryptophane (Trp) ou du 5-hydroxytryptophane (5-HTP), ledit procédé comprenant les étapes suivantes :
1) la préparation du mélange de la couche "rapide" par les sous-étapes suivantes :
i) la granulation d'un mélange des ingrédients suivants : une première partie de cellulose microcristalline, une première partie de croscarmellose sodique, du 5-hydroxytryptophane (5-HTP), de l'hydroxypropylméthylcellulose et un colorant, avec une solution de granulation comprenant de l'eau déminéralisée et de la polyvinylpyrrolidone ;
ii) le dimensionnement du granulat ;
iii) le séchage du granulat dimensionné dans un four sous vide à 45°C jusqu'à une teneur en humidité du granulat inférieure à 1 % ;
iv) le tamisage du granulat séché à travers un tamis d'environ 2 mm ;
v) l'addition, au granulat tamisé, de la partie restante de croscarmellose sodique, de stéarate de magnésium, de phosphate dicalcique et de polyvinyl-polypyrrolidone, tamisés à travers un tamis d'une maille d'environ 0,7 mm ;
vi) le mélange du produit de la sous-étape v) ;
2) la préparation du mélange de la couche "retard" par les sous-étapes suivantes :
a) le mélange d'un ingrédient parmi le tryptophane et le 5-hydroxytryptophane avec du phosphate dicalcique ;
b) la granulation du mélange de la sous-étape a) avec une solution de granulation comprenant de l'eau déminéralisée et de la polyvinyl-polypyrrolidone ;
c) le dimensionnement du granulat de la sous-étape b) et le séchage dans un four sous vide à 45°C jusqu'à une teneur en humidité inférieure à 1 % ;
d) l'addition, au produit précédemment tamisé de la sous-étape c), de vitamines, de talc, de stéarate de magnésium, de dioxyde de silicium, de cellulose microcristalline, de polyvinyl-polypyrrolidone et d'hydroxypropylméthylcellulose, tamisés à travers un tamis d'une maille d'environ 0,7 mm ;
4) l'alimentation des deux mélanges des étapes 1) et 2) dans une machine à former des comprimés convenables.

2. Procédé selon la revendication 1, dans lequel la sous-étape 2) a) comprend l'utilisation de L-Trp.

3. Procédé selon la revendication 1 ou 2, dans lequel une étape 3) est proposée de mélange de cellulose microcristalline, de phosphate dicalcique, de stéarate de magnésium végétal et de polyvinyl-polypyrrolidone, et dans lequel l'étape 4), en plus de l'alimentation des deux mélanges des étapes 1) et 2) dans une machine à former des comprimés convenable, propose aussi l'alimentation de celui de l'étape 3) dans celle-ci.

4. Procédé selon l'une quelconque des revendications 1 à 3, dans lequel le colorant de la sous-étape 1) i) est le colorant E132.

5. Procédé selon l'une quelconque des revendications 1 à 4, dans lequel le comprimé obtenu dans l'étape 4) après 24 mois de stockage présente une libération de plus de 90 % du 5-HTP de la couche "rapide" en l'espace de 5 minutes après l'administration, d'environ 50 à 70 % du Trp ou du 5-HTP de la couche "retard" en l'espace de 4 heures et de plus de 80 % du Trp ou du 5-HTP en l'espace de 8 heures de la couche "retard".

6. Comprimé à deux couches, dans lequel une couche est "rapide" et comprend les ingrédients suivantes :
| |
|---|
| 5-HTP |
| cellulose microcristalline |
| phosphate dicalcique |
| hydroxypropylméthylcellulose |
| croscarmellose sodique |
| polyvinylpyrrolidone |
| stéarate de magnésium végétal |
| polyvinyl-polypyrrolidone |
| colorant E132 |
et l'autre couche est "retard" et comprend les ingrédients suivantes :
| |
|---|
| phosphate dicalcique |
| L-tryptophane |
| cellulose microcristalline |
| vitamines |
| hydroxypropylméthylcellulose |
| polyvinylpyrrolidone |
| stéarate de magnésium végétal |
| dioxyde de silicium |
| talc |
| polyvinyl-polypyrrolidone |

7. Comprimé à deux couches, dans lequel une couche est "rapide" et comprend les ingrédients suivantes :
| |
|---|
| 5-HTP |
| cellulose microcristalline |
| phosphate dicalcique |
| hydroxypropylméthylcellulose |
| croscarmellose sodique |
| polyvinylpyrrolidone |
| stéarate de magnésium végétal |
| polyvinyl-polypyrrolidone |
| colorant E132 |
et l'autre couche est "retard" et comprend les ingrédients suivantes :
| |
|---|
| phosphate dicalcique |
| 5-HTP |
| cellulose microcristalline |
| vitamines |
| hydroxypropylméthylcellulose |
| polyvinylpyrrolidone |
| stéarate de magnésium végétal |
| dioxyde de silicium |
| talc |
| polyvinyl-polypyrrolidone |

8. Comprimé à trois couches, dans lequel une couche est "rapide" et comprend :
| |
|---|
| 5-HTP |
| cellulose microcristalline |
| phosphate dicalcique |
| hydroxypropylméthylcellulose |
| croscarmellose sodique |
| polyvinylpyrrolidone |
| stéarate de magnésium végétal |
| polyvinyl-polypyrrolidone |
| colorant E132 |
une couche qui est une couche inerte qui comprend :
| |
|---|
| cellulose microcristalline |
| phosphate dicalcique |
| polyvinyl-polypyrrolidone |
| stéarate de magnésium végétal |
et une couche est une couche "retard" qui comprend :
| |
|---|
| phosphate dicalcique |
| L-tryptophane |
| cellulose microcristalline |
| polyvinyl-polypyrrolidone |
| vitamines |
| hydroxypropylméthylcellulose |
| polyvinylpyrrolidone |
| stéarate de magnésium végétal |
| dioxyde de silicium |
| talc |

9. Comprimé selon l'une quelconque des revendications 6 à 8, à utiliser dans le traitement de pathologies ou d'états qui mettent en jeu une carence en sérotonine.

10. Comprimé selon la revendication 9, dans lequel les carences en sérotonine se produisent dans le système nerveux central.
